# EUROPÄISCHE PATENTSCHRIFT

(11) **EP 1 498 073 B1**
(45) Veröffentlichungstag und Bekanntmachung des Hinweises auf die Patenterteilung: **02.04.2008**
(21) Anmeldenummer: 03405537.6
(22) Anmeldetag: 15.07.2003
(51) Int. Cl.: A61B 17/00, A61M 5/19, A61J 1/00, B65D 81/32

(54) **System und Verfahren zum Mischen von wenigstens vier Komponenten**
System and procedure for mixing of at least four components
Système et procédé pour mélanger au minimum quatre composants

(43) Veröffentlichungstag der Anmeldung: 19.01.2005
(73) Patentinhaber: Straumann Holding AG, 4002 Basel (CH)
(72) Erfinder: Fehr, Daniel, 8008 Zürich (CH); Neidhardt, Astrid, 8001 Zürich (CH); Damm, Christian, 4450 Sissach (CH)
(74) Vertreter: Schaad, Balass, Menzl & Partner AG

(56) Entgegenhaltungen:
- EP-A- 0 292 472
- DE-C- 19 636 622
- FR-A- 2 412 475
- US-A1- 2001 037 091
- US-A1- 2002 198 490

## Beschreibung

Die Erfindung betrifft ein System und ein Verfahren zum Mischen von wenigstens vier Komponenten mit den Merkmalen des Oberbegriffs der unabhängigen Patentansprüche.

Bei vielen Anwendungen, insbesondere im medizinischen Bereich ist es erforderlich, kurz vor der Applikation verschiedene Komponenten miteinander zu vermischen. Dies können insbesondere Komponenten einer Zweikomponenten-Zusammensetzung sein, die bei Kontakt miteinander aushärten, beispielsweise Zahnfüllmassen.

Solche Komponenten sind häufig beispielsweise aus hygienischen Gründen in Einwegspritzen verpackt. Aus US 5 116 315, US 4 735 616, US 6 328 229 oder US 2002/0042591 sind beispielsweise Mischanordnungen bekannt, bei denen die in zwei Spritzen enthaltenen Komponenten gemeinsam durch einen Mischer ausgegeben und dabei gemischt werden.

Ausserdem ist es bekannt, zwei Spritzen an ihren Enden miteinander zu verbinden, so dass durch Hin- und Hertransferieren der in den Spritzen enthaltenen Komponenten die Komponenten durchmischt werden können. Solche Anordnungen sind beispielsweise aus US 4 743 229, US 4 994 029, US 6 234 196 oder US 2002/0055708 bekannt.

Eine derartige Spritzenanordnung gemäß dem Oberbegriff des Anspruchs 1 ist ebenfalls in der EP-B-0292472 offenbart. Die dortige Spritzenanordnung weist vier Spritzenkörper auf, von denen jeweils zwei in einer gemeinsamen Haltevorrichtung gehalten sind. Spritzenkörper der ersten Haltevorrichtung sind dabei mit Lyophilisaten, Spritzenkörper der zweiten Haltevorrichtung mit Lösungsmitteln gefüllt. Über eine direkte Verbindung von Konussen der Spritzenkörper bzw. über ein Kupplungsstück können die Inhalte der Spritzenkörper miteinander vermischt werden.

Des weiteren ist aus DE-C-19 636 622 ein Halter für die Aufnahme der Spritzen gemäß dem Oberbegiff des Anspruchs 13 bekannt.

Ein Nachteil bei diesen.bekannten Anordnungen besteht darin, dass sich mehr als zwei Komponenten überhaupt nicht oder nur mit erhöhtem Aufwand miteinander vermischen lassen. Insbesondere bei biomedizinischen Anwendungen ist es aber häufig wünschenswert, zwei Komponenten, beispielsweise zwei Polymere zuerst mit Puffern zu vermischen und anschliessend zur Vernetzung oder Polymerisation miteinander in Kontakt zu bringen. Die Polymere und wässrigen Lösungen werden dabei häufig auf Grund der Stabilität separat von einander gelagert.

Es ist deshalb die Aufgabe der vorliegenden Erfindung, die Nachteile des Bekannten zu vermeiden, insbesondere also eine Vorrichtung und ein Verfahren zum Mischen von wenigstens vier Komponenten zu schaffen, welches ein Vertauschen der Spritzen Verhindert. Das System soll auf einfache Art und Weise und kostengünstig herstellbar sein. Ausserdem soll eine hygienische Verarbeitung der Komponenten, insbesondere eine kontaminationsfreie Mischung möglich sein.

Erfindungsgemäss werden diese Aufgaben mit einer Vorrichtung und mit einem Verfahren mit den Merkmalen der characterisienden Teile der unabhängigen Patentansprüche gelöst.

Die erfindungsgemäße Vorrichtung dient zum Mischen von wenigstens vier Komponenten. Hier und im Folgenden wird die Erfindung an Hand von vier Komponenten erläutert. Das gleiche System könnte aber grundsätzlich auch zum Mischen von mehr, beispielsweise 6 Komponenten verwendet werden. Die Vorrichtung weist eine erste und eine zweite Spritzenanordnung auf. In der ersten und in der zweiten Spritzenanordnung sind je wenigstens zwei Kammern zur Aufnahme von je einer Komponente enthalten. Es ist also jede der vier Komponenten in einer von zwei Kammern einer der beiden Spritzenanordnungen enthalten. Jede Kammer der einen Spritzenanordnung ist lösbar mit einer Kammer der anderen Spritzenanordnung verbunden, so dass jede in einer Kammer der einen Spritzananordnung enthaltene Komponente in eine Kammer der anderen Spritzenanordnung transferierbar und mit der in dieser Kammer enthaltenen Komponente mischbar ist. Mit dieser Vorrichtung lassen sich in einem ersten Schritt durch Transfer von der einen Spritzenanordnung in die andere Spritzenanordnung je zwei Komponenten mit je zwei anderen Komponenten vermischen, so dass zwei erste Gemische gebildet werden. Diese ersten Gemische sind nach dem Transfer in den Kammern einer Spritzenanordnung enthalten. Diese ersten Gemische können anschliessend aus dieser Spritzenanordnung abgegeben und untereinander vermischt werden, so dass sich ein zweites Gemisch aus allen vier Komponenten ergibt.

Gemäss einer Ausführungsform weist daher die Vorrichtung ausserdem eine Mischanordnung auf. Die Mischanordnung ist typischerweise ein statischer Mischer. Die Mischanordnung ist mit den Kammern einer der Spritzenanordnungen so verbindbar, dass die in den Kammern enthaltenen Gemische durch Abgabe aus den Kammern und durch die Mischanordnung weiter miteinander mischbar sind. Mit einer solchen Vorrichtung lassen sich in zwei Schritten zuerst je zwei Komponenten miteinander vermischen. Durch Verbindung der einen Spritzenanordnung, in der die Gemische enthalten sind mit einer Mischanordnung lassen sich diese Gemische anschliessend in einem weiteren Schritt zum gewünschten Gemisch vermischen. Das Handling ist sehr einfach, weil die als Behälter für die Komponenten dienenden Spritzenanordnungen zweimal zum Mischen eingesetzt werden.

Um zu verhindern, dass die ersten und zweiten spritzenanordnungen versehentlich falsch zusammengefügt werden, kann die Vorrichtung in einer bevorzugten Ausführungsform Mittel aufweisen, mit denen eine eindeutige, das heisst nur eine einzige mögliche Verbindung der Spritzenanordnungen untereinander sichergestellt ist. Denkbar ist beispielsweise eine Kodierhülse an der einen Spitzenanordnung, in welche ein Vorsprung an der anderen Spritzenanordnung in nur genau einer Stellung einsetzbar ist.

Gemäss einer weiteren bevorzugten Ausführungsform weist ausserdem wenigstens eine der Spritzenanordnungen Spritzenkolben auf, welche miteinander verbindbar sind. Typischerweise können die Kolben miteinander zusammengesteckt oder zusammengeschnappt werden. Mit einer solchen Verbindung wird sichergestellt, dass die in den beiden Kammern enthaltenen Komponenten gleichmässig ausgestossen werden. Durch den gleichmässigen Ausstoss der Komponenten in den beiden Kammern wird auch eine gleichmässige Durchmischung beim Transfer in die Kammern der anderen Spritzenanordnung und/oder bei der Ausgabe der ersten Gemische durch die Mischanordnung erzielt.

Besonders bevorzugt bestehen die Spritzen der einen Spritzenanordnung aus Glas, während die Spritzen der anderen Spritzenanordnung aus Kunststoff bestehen. Typischerweise befinden sich in den Kammern der aus Glas gebildeten Spritzenanordnungen Polymere wie zum Beispiel PEG-Acrylate, PEG-Thiol zur Bildung von Hydrogelen und zum Einsatz als Membrane, welche das Einwachsen von Gewebe am applizierten Ort verhindert. In den aus Kunststoff gebildeten Spritzen befinden sich typischerweise Flüssigkeiten wie zum Beispiel wässrige Carboxy-Methyl-Cellulose-Lösung (CMC-Lösung) mit bzw. ohne Puffersalzen, gegebenenfalls mit Katalysatoren. Bei Einsatz von Puffern in diesen Flüssigkeiten sind insbesondere basische Puffer bevorzugt.

Bevorzugt sind die Spritzen als herkömmliche Spritzen ausgebildet, die beim Einsetzen in einen Halter zu den beschriebenen Spritzenanordnungen zusammengesetzt werden können.

Die Verbindungsanordnung zwischen der ersten und der zweiten Spritzenanordnung ist ausserdem bevorzugt so ausgebildet, dass die Verbindung nur dann lösbar ist, wenn die gemischten Komponenten in den Kammern der einen Spritzenanordnung befinden. Insbesondere sollen sich die ersten Gemische in der aus Glas gebildeten Spritzenanordnung befinden. Damit ist sichergestellt, dass die Spritzenanordnungen erst nach korrektem Transfer der Komponenten von der einen Spritzenanordnung in die andere Spritzenanordnung wieder voneinander getrennt werden. Die Mischanordnung kann daher erst aufgesetzt werden, wenn sich die Gemische in der gewünschten Spritzenanordnung befinden. Dazu kann z.B. eine Einrastanordnung vorgesehen werden, die sich nur bei zurückgeschobenen Kolben der Spritzen lösen lässt.

Die lösbare Verbindung zwischen den Spritzenanordnungen ist bevorzugt als Schnappverbindung ausgebildet. Auf diese Weise lassen sich die beiden Spritzenanordnungen auf einfache Weise und in einer klar definierten Endlage miteinander verbinden. Selbstverständlich sind aber auch andere Verbindungsmechanismen denkbar. Die Mischanordnung lässt sich bevorzugt ebenfalls mit einer Schnappverbindung mit der einen Spritzenanordnung verbinden. Es ist denkbar aber nicht zwingend, dass für die Verbindung zwischen den Spritzenanordnungen einerseits und für die Verbindung zwischen der einen Spritzenanordnung und der Mischanordnung die gleichen Verbindungsmittel verwendet werden.

Um den Mischvorgang bei der Abgabe der Gemische durch die Mischanordnung zu überwachen ist es ausserdem denkbar, die Mischanordnung wenigstens teilweise transparent auszubilden. Durch eine Art Sichtfenster lässt sich dabei der Mischvorgang überwachen.

Gemäss einer weiteren bevorzugten Ausführungsform kann die Mischanordnung ausserdem eine gebogene Spitze aufweisen. Dadurch erleichtert man das Anbringen in schwer zugänglichen Orten, z.B. im oralen Bereich.

Der erfindungsgemässe Halter dient zur Aufnahme von wenigstens zwei Spritzen und ist zur Verbindung mit einem zweiten Halter ausgebildet.

Das erfindungsgemässe Verfahren dient zum Mischen von wenigstens vier Komponenten. In dem Verfahren werden zuerst Komponenten in je wenigstens zwei Kammern von zwei Spritzenanordnungen bereitgestellt. Diese Kammern sind je mit Ausgabeöffnungen versehen. Anschliessend werden die Ausgabeöffnungen je einer Kammer der einen Spritzenanordnung mit je einer Kammer der anderen Spritzenanordnung verbunden wobei mittels Codiermitteln in form einer Codierhülse oder einer Codierscheibe ein Vertauschen der Spritzenanordnungen Verhindert wird. Danach werden die Komponenten aus den Kammern der ersten Spritzenanordnung in je eine Kammer der zweiten Spritzenanordnung transferiert. Der Transfer erfolgt typischerweise durch Ausstossen der Komponenten mittels einem Kolben der Spritze. Beim Transfer werden die transferierten Komponenten mit den in der zweiten Spritzenanordnung enthaltenen Komponenten vermischt. Dabei werden erste Gemische gebildet. In einem letzten Schritt werden die in den Kammern enthaltenen ersten Gemische abgegeben und unter weiterer Mischung miteinander zum Bilden eines zweiten Gemisches vermischt.

Gemäss einem bevorzugten Ausführungsbeispiel des erfindungsgemässen Verfahrens werden die durch einen erstmaligen Transfer vermischten Komponenten zwecks weiterer Vermischung aus den Kammern der zweiten Spritzenanordnung in die Kammern der ersten Spritzenanordnung zurück transferiert.

Gemäss einem weiteren bevorzugten Ausführungsbeispiel werden die derart gebildeten ersten Gemische durch einen statischen Mischer abgegeben und dabei zu den zweiten Gemischen vermischt.

Die Erfindung wird im Folgenden an Hand der Zeichnungen und in Ausführungsbeispielen näher erläutert. Es zeigen:
- Figur 1:: Schematische Darstellung eines Ausführungsbeispiels von zwei nicht erfindungsgemässen Spritzenanordnungen,
- Figur 2:: schematische Darstellung eines nicht erfindungsgemässen statischen Mischers,
- Figur 3a, b, c:: schematische Darstellung des nicht erfindungsgemässen Verfahrens zum Mischen von Komponenten,
- Figur 4:: Explosionszeichnung des Verbindungsmechanismus einer ersten Spritzenanordnung eines ersten Ausführungsbeispiels,
- Figur 5a bis 5c:: detaillierte dreidimensionale Darstellung einer ersten Spritzenanordnung in einer ersten Ausführungsform, die Verriegelung und Entriegelung einer zweiten Spritzenanordnung sind als Schnitt parallel zur Bewegungsachse des Verbindungsfortsatzes dargestellt,
- Figur 6a bis 6d:: schematische Darstellung des Mischvorgangs der Ausführungsform gemäss Figuren 5a bis 5c in dreidimensionaler Form,
- Figur 7:: Explosionszeichnung des Verbindungsmechanismus einer ersten Spritzenanordnung eines alternativen Ausführungsbeispiels,
- Figuren 8a, 8b:: detaillierte Darstellung des Verriegelungs- und Entriegelungsmechanismus der Verbindungsanordnung gemäss Figur 7 und
- Figur 9a bis 9d:: schematische Darstellung des Mischvorgangs des Ausführungsbeispiels gemäss Figur 7 in perspektivischer Darstellung.

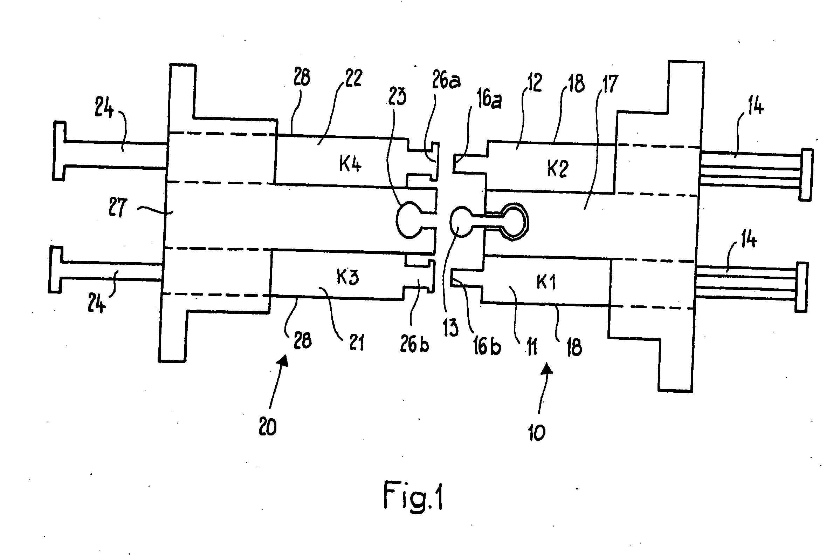

Figur 1 zeigt schematisch eine erste Spritzenanordnung 10 und eine zweite Spritzenanordnung 20. Die erste Spritzenanordnung 10 weist zwei Spritzen 18 auf, welche in einem Halter 17 eingesetzt und dadurch miteinander verbunden sind. Die beiden Spritzen 18 weisen je eine Kammer 11, 12 auf. In den Kammern 11, 12 sind Komponenten K1, K2 enthalten.

Die zweite Spritzenanordnung 20 weist ebenfalls zwei Spritzen 28 auf, die über einen Halter 27 miteinander verbunden sind. Die Spritzen 28 weisen Kammern 21, 22 zur Aufnahme von Komponenten K3 und K4 auf.

Die Spritzen 18 bzw. 28 sind in an sich bekannter Weise aufgebaut und weisen Kolben 14 bzw. 24 zur Ausgabe der in den Spritzen enthaltenen Komponenten K1, K2, K3, K4 durch Ausgabeöffnungen 16a, 16b bzw. Ausgabeöffnungen 26a, 26b auf. Die Spritzen 18 der ersten Spritzenanordnung 10 können typischerweise als Glasspritzen ohne Luer-Lock des Herstellers Becton Dickinson sein. Die Spritzen 28 der zweiten Spritzenanordnung 20 sind typischerweise Plastikspritzen mit einem male Inlet von Ultradent.

Die erste Spritzenanordnung 10 weist einen Nocken 13 auf, der in eine Aussparung 23 an der zweiten Spritzenanordnung 20 einschnappbar ist. Mit der Aussparung 23 und dem Nocken 13 lassen sich die ersten Spritzenanordnung 10 und die zweite Spritzenanordnung 20 so miteinander verbinden, dass die Ausgabeöffnungen 16a, 26a bzw. 16b, 26b der Spritzen 18, 28 je miteinander verbunden sind.

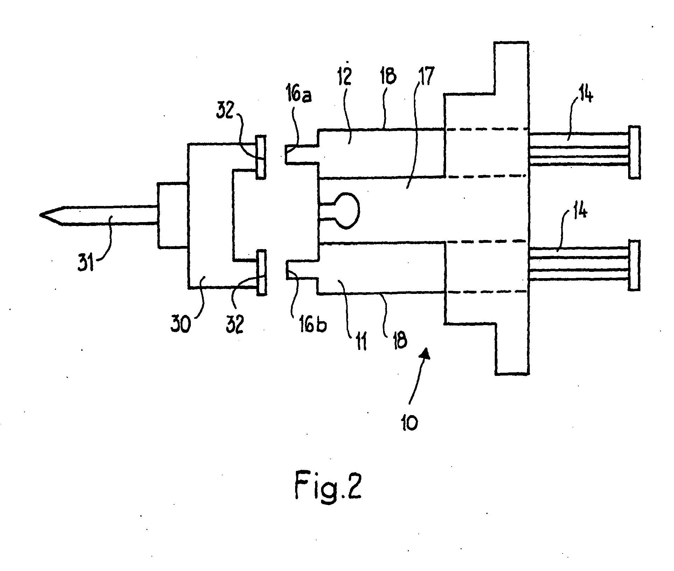

Das System weist ausserdem eine Mischvorrichtung 30 auf. In Figur 2 ist die als statischer Mixer ausgebildete Mischvorrichtung 30 schematisch dargestellt. Die Mischvorrichtung 30 weist zwei Öffnungen 32 auf, welche sich mit den Ausgabeöffnungen 16a, 16b der ersten Spritzenanordnung 10 verbinden lassen. Die Mischvorrichtung 30 ist ausserdem mit einer Spitze 31 versehen, mittels welcher sich in der Mischvorrichtung 30 gemischte Komponente abgeben lassen.

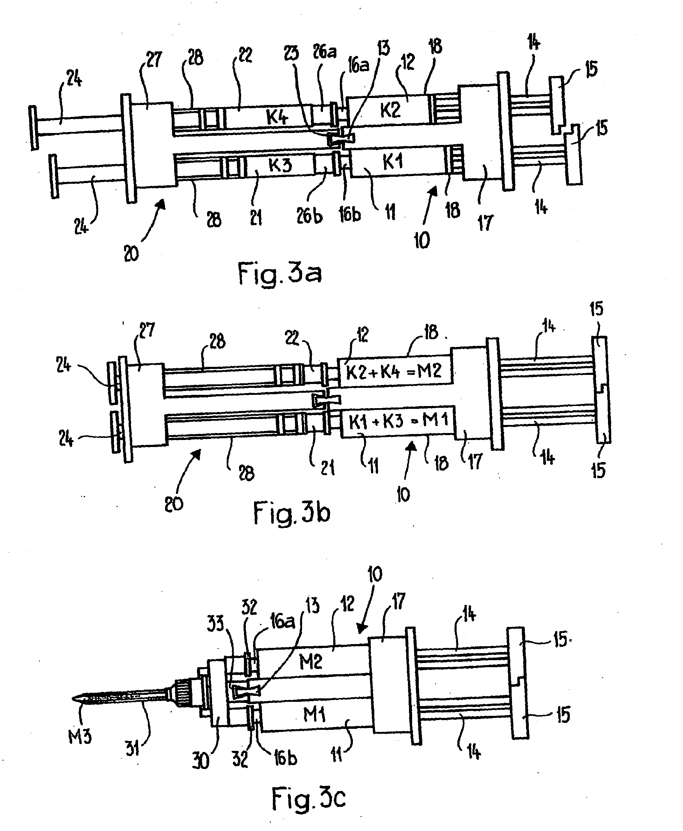

Der Mischvorgang mit der Mischvorrichtung 30 ist schematisch in den Figuren 3a bis 3c gezeigt.

Mit dem System bzw. Verfahren sollen vier Komponente K1, K2, K3 und K4 miteinander vermischt werden. Bei - den Komponenten handelt es sich typischerweise um Puffer wie z.B. CMC mit Wasser oder verschiedenen basischen Puffern sowie PEG-Acrylate oder PEG-Thiol.

Die Komponenten sind je in Spritzen 18 bzw. 28 enthalten. Zwei Spritzen 18 bzw. 28 werden mit einem Halter 17 bzw. 27 zu einer ersten Spritzenanordnung 10 bzw. zu einer zweiten Spritzenanordnung 20 verbunden. Dieses Verbinden kann bereits Teil des Mischverfahrens sein. Es ist aber auch denkbar, die Spritzenanordnungen 10, 20 mit je zwei Spritzen vormontiert bereitzustellen.

Die Halter 17, 27 sind so ausgebildet, dass sich die nicht gezeigten Kappen der Spritzen, insbesondere der spritzen 28 durch einfaches Drehen entfernen lassen. Insbesondere soll der Halter 27 diese Drehbewegung nicht behindern.

In einem ersten Schritt des Mischverfahrens werden die Spritzenanordnungen 10, 20 mit ihren Ausgabeöffnungen 16a, 16b bzw. 26a, 26b miteinander dichtend verbunden. Dazu dient eine Haltevorrichtung mit Nocken 13 und Aussparung 23, mittels welcher sich die beiden Halter 17, 27 der Spritzenanordnungen 10, 20 schnappbar miteinander verbinden lassen.

Zum Mischen der Komponenten werden zuerst die Komponenten K1, K2 aus den Kammern 11, 12 der ersten Spritzenanordnung 10 in die Kammern 21, 22 der zweiten Spritzenanordnung 20 transferiert. Dies erfolgt durch Betätigen der Kolben 14 der ersten Spritzenanordnung 10, so dass die Komponenten K1, K2 durch die Ausgabeöffnungen 16b, 16a in die Kammern 21, 22 der zweiten Spritzenanordnung 20 gegeben werden. Bei diesem Vorgang vermischen sich die Komponenten K1, K2 jeweils mit den bereits in den Kammern 21, 22 der zweiten Spritzenanordnung 20 enthaltenen Komponenten K3, K4. Nach diesem Transferschritt sind die Kammern 11, 12 der ersten Spritzenanordnung 10 leer und in den Kammern 21, 22 der zweiten Spritzenanordnung 20 befinden sich aus den Komponenten K1 und K3 - bzw. aus den Komponenten K2 und K4 bestehende Gemische M1 und M2. Dieser Zustand ist in den Figuren 3a bis 3c nicht dargestellt. Zur besseren Verbesserung der Durchmischung der Gemische M1, M2 werden diese Gemische anschliessend von der zweiten Spritzenanordnung 20 in die erste Spritzenanordnung 10 zurücktransferiert.

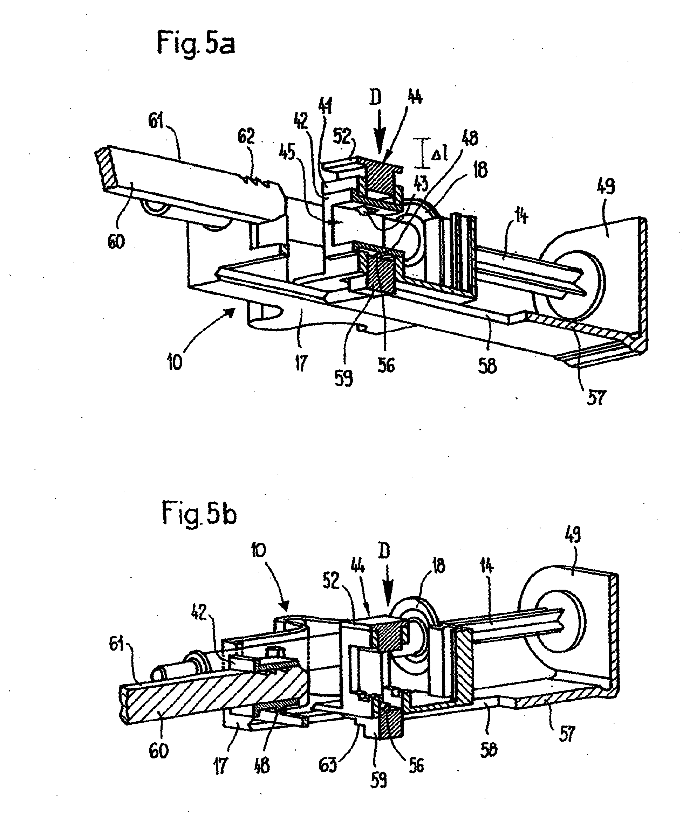

Figur 3b zeigt die zurücktransferierten Gemische M1, M2. Die Kammern 21, 22 der zweiten Spritzenanordnung 20 sind leer. In diesem Zustand ist es möglich, die erste Spritzenanordnung 10 wieder von der zweiten Spritzenanordnung 20 zu trennen. (siehe Beschreibung zur Figur 5b). An Stelle der zweiten Spritzenanordnung 20 wird in einem nächsten Schritt (siehe Figur 3c) die Mischvorrichtung 30 mit den Ausgabeöffnungen 16a, 16b der ersten Spritzenanordnung 10 verbunden. Dazu weist die Mischvorrichtung 30 Öffnungen 32 auf, die dichtend mit den Ausgabeöffnungen 16a, 16b zusammenpassen. Ausserdem weist die Mischvorrichtung 30 eine Schnappvorrichtung 33 auf, welche sich lösbar schnappend mit dem einen Vorsprung bildenden Nocken 13 der ersten Spritzenanordnung 10 verbinden lässt. Durch Drücken der Kolben 14 der ersten Spritzenanordnung 10 werden die in den Kammern 11, 12 enthaltenen Gemische M1, M2 durch die Mischvorrichtung 30 transferiert und dabei miteinander zu einem Gemisch M3 vermischt, das durch die Spitze 31 der Mischvorrichtung 30 abgegeben werden kann.

Die Mischvorrichtung 30 ist in herkömmlicher Art und Weise aufgebaut.

Um sicherzustellen, dass während dem jeweiligen Transfer die Abgabe der Komponenten gleichmässig erfolgt, ist die erste Spritzenanordnung 10 mit einer Verbindungsvorrichtung 15 versehen. Mittels zweier auf die Kolben 14 eingesetzten Clips lassen sich die Kolben miteinander verbinden. Dadurch ist ein gleichmässiger Ausstoss der Gemische M1, M2 durch die Mischvorrichtung 30 aber auch bereits ein gleichmässiger Transfer der Komponenten K1, K2 (siehe Figur 3a) in die Kammern 21, 22 der zweiten Spritzenanordnungen 20 sichergestellt.

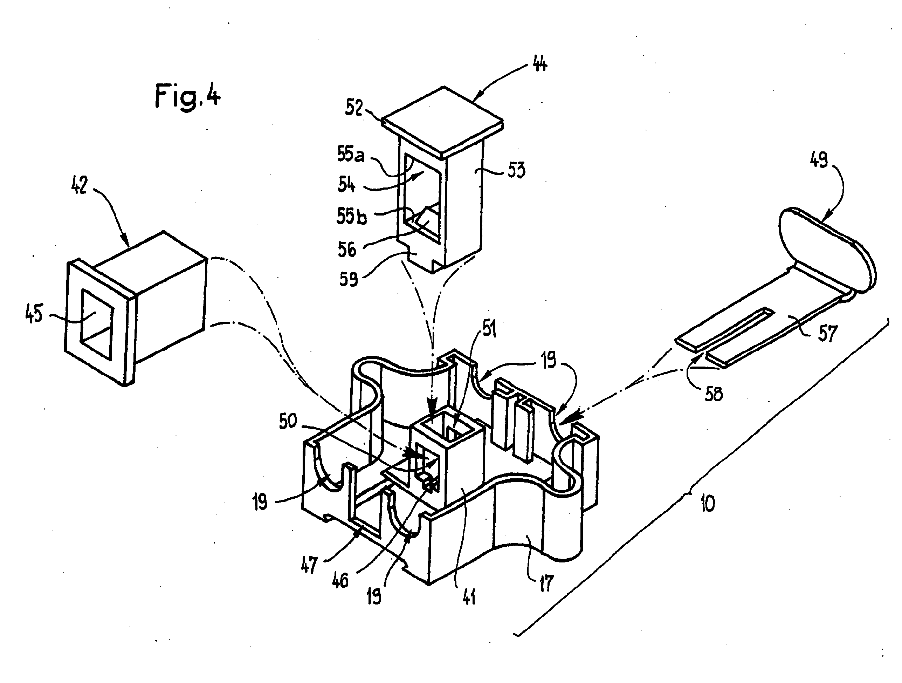

Figur 4 zeigt schematisch in Form einer Explosionsdarstellung eine Ausführungsform der ersten Spritzenanordnung 10 mit einem Verbindungsmechanismus, der ein Lösen der beiden Spritzenanordnungen 10, 20 vor dem Mischen verhindert. Ähnlich wie in den vorangehenden Figuren 2 und 3 werden Spritzen 18 in Spritzenaufnahmen 19 des Halters 17 eingesetzt. Der Halter 17 ist ausserdem am stirnseitigen Ende zwischen den Spritzenaufnahmen 19 mit einer Einführöffnung 47 zum Durchführen eines Verbindungselementes (siehe nachfolgende Figuren 5a bis 5c) versehen. Der Halter 17 weist eine Halterung 41 zur Aufnahme eines Entriegelungsknopfes 44 auf, mittels welchem sich die ersten und zweiten Spritzenanordnungen 10, 20 nach dem Mischen wieder voneinander trennen lassen. Der Entriegelungsknopf 44 ist in eine Längsöffnung 51 der Halterung 41 verschiebbar eingesetzt. Die Halterung 41 weist ausserdem eine Öffnung 50 mit einem rechteckigen Querschnitt auf. In die Öffnung 50 wird eine Codierhülse 42 eingesetzt. Am unteren Rand der Öffnung 50 ist eine Aussparung 46 ausgeformt, welche eine Führung für einen Verbindungsfortsatz 34 der Mischvorrichtung 30 bildet.
Die Codierhülse 42 enthält eine Öffnung 45 zur Einführung eines Verbindungsfortsatzes 60 (nicht gezeigt). Nicht erkennbar in der Figur 4 ist eine Rastlasche 43, welche an einer inneren, oberen Begrenzungsfläche einer Öffnung 45 der Codierhülse 42 angeordnet ist und mit Sägezähnen 62 des Verbindungsfortsatzes 60 in Eingriff gelangt (siehe Figur 5a).
Der Entriegelungsknopf 44 weist einen Kopfteil 52 und einen Körperteil 53 auf. Im Körperteil 53 ist eine Vertiefung 54 zur Aufnahme der Codierhülse 42 vorgesehen. Die Vertiefung 54 ist durch eine obere Begrenzungsfläche 55a und eine untere Begrenzungsfläche 55b definiert. Auf der unteren Begrenzungsfläche 55b ist ein Rastnocken 56 angeordnet, welcher einen Rastnocken der Codierhülse 42 zurückhält.
Die Kolben 14 zweier in den Halter 17 eingelegten Spritzen 18 werden durch eine Verbindungsplatte 49 miteinander verbunden. Die Verbindungsplatte 49 weist eine Verlängerung 57 sowie eine Nut 58 auf. Die Nut 58 ist so ausgebildet, dass sie formschlüssig mit einem Vorsprung 59 des Entriegelungsknopfes 44 ist.

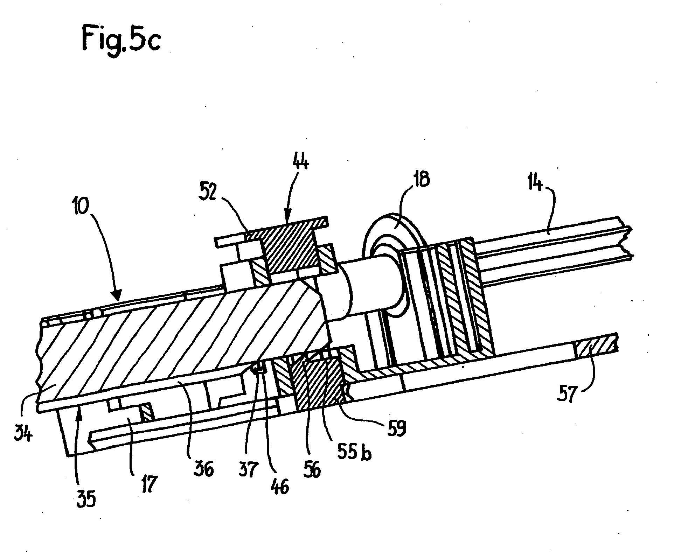

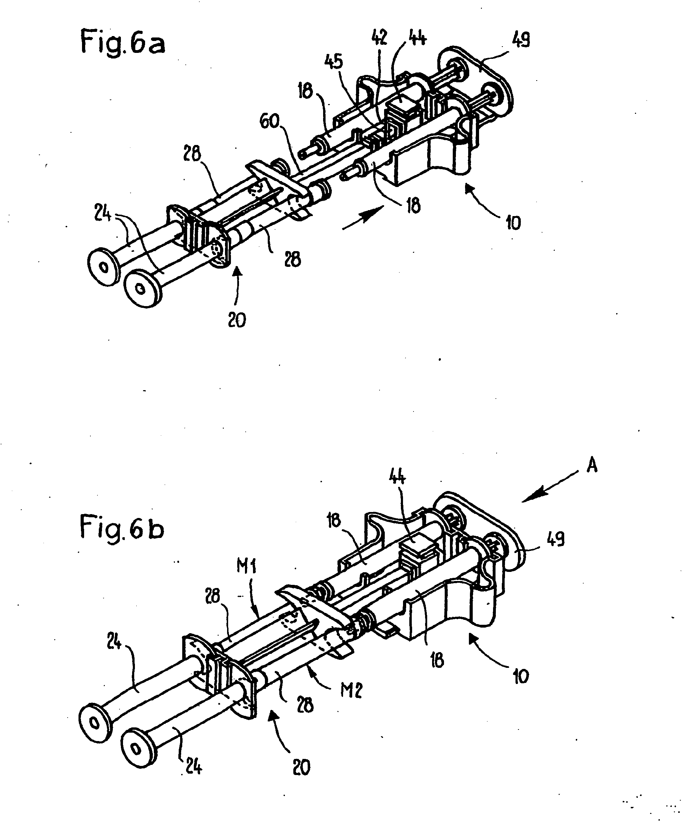

Die Funktion der Codierhülse 42 wird nun an Hand der Figuren 5a, 5b und 5c genauer beschrieben. Die zweite Spritzenanordnung 20 weist den Verbindungsfortsatz 60 auf. In Figur 5a ist nur der Verbindungsfortsatz 60 gezeigt, der in geeigneter Weise (siehe beispielsweise Figur 6a bis 6c) mit der zweiten Spritzenanordnung verbunden ist. Der Verbindungsfortsatz 60 der zweiten Spritzenanordnung 20 ist in die im Querschnitt vorzugsweise rechteckige Öffnung 45 in der Codierhülse 42 einführbar. Auf seiner Oberseite 61 weist der Verbindungsfortsatz 60 die Sägezähne 62 auf. Mittels der Sägezähne 62 ist der Verbindungsfortsatz 60 an der federnden Rastlasche 43 an der oberen Wand der Öffnung 45 der Codierhülse 42 einrastbar. Wenn der Verbindungsfortsatz 60 einmal in die Codierhülse 42 eingesteckt ist, kann der Fortsatz 60 auf Grund der Verrastung der Rastlasche 43 mit den Sägezähnen 62 nicht mehr einfach aus der Codierhülse 42 entfernt werden. Die Halterung 41 ist ausserdem in einer Richtung senkrecht zu der Öffnung 50 für die Codierhülse 42 mit der Längsöffnung 51 zur Aufnahme des Entriegelungsknopfes 44 versehen.

Der-Entriegelungsknopf 44 ist in der Richtung der Längsöffnung 51 um eine Distanz Δl verschiebbar. In Figur 5a ist der Entriegelungsknopf 44 in einer oberen Stellung gezeigt, in der die Codierhülse 42 auf Grund des Zusammenwirkens zwischen einem Rastnokken 48 und dem Rastnocken 56 in der Halterung 41 gehalten wird. Durch Herunterdrücken des Entriegelungsknopfes 44 in Pfeilrichtung D gelangt die Rastnocke 56 am Entriegelungsknopf 44 ausser Eingriff mit der Rastnocke 48 der Codierhülse 42. Dadurch kann die Codierhülse 42 und damit auch der in der Codierhülse 42 eingerastete Verbindungsfortsatz 60 aus der Halterung 41 entfernt werden.

In Figuren 5a und 5b ist auch die Anordnung gezeigt, die sicherstellt, dass der Verriegelungsknopf 44 nur in Pfeilrichtung D nach unten gedrückt werden kann, wenn die Kolben 14 der Spritzen - 18 sich in geöffneter Stellung befinden. Auf diese Weise wird sichergestellt, dass die erste und die zweite Spritzenanordnung 10, 20 nur dann voneinander getrennt werden können, wenn die Kolben 14 der ersten Spritzenanordnung 10 in der geöffneten Stellung sind, das heisst nur wenn die durch das Hin- und Hertransferieren vermischte Flüssigkeit sich in den Spritzen 18 der ersten Spritzenanordnung 10 befinden.

Figur 5a zeigt, dass die Verbindungsplatte 49, welche die Kolben 14 miteinander verbindet, die auf der Unterseite des Halters 17 verschiebbar angeordnete Verlängerung 57 aufweist. Die Verlängerung 57 weist die Nut 58 auf. In der Nut 58 ist (siehe Figur 5a) der auf der Unterseite des Entriegelungsknopfs 44 angeordneter Vorsprung 59 geführt. Wenn die Kolben 14 in die Spritzen 18 hineingedrückt sind, liegt der Vorsprung 59 des Entriegelungsknopfs 44 in der Nut 58 der Verlängerung 57. Die Unterseite 63 des Verriegelungsknopfs 44 liegt dabei auf der Oberseite der Verlängerung 57-auf. Dadurch lässt sich der Verriegelungsknopf 40 nicht mehr in Pfeilrichtung D nach unten drücken. Wenn die Kolben 14 sich in der geöffneten Stellung befinden (siehe Figur 5b), wird die Unterseite 63 des Entriegelungsknopfs 44 freigegeben. Dann lässt sich der Entriegelungsknopf 44 in Pfeilrichtung D drücken und die Codierhülse 42 lässt sich mit darin eingesetztem Verbindungsfortsatz 60 entfernen.

Figur 5c zeigt die Verrastung des Verbindungsfortsatzes 34 der Mischvorrichtung 30 im Entriegelungsknopf 44. Der Verbindungsfortsatz 34 der Mischvorrichtung 30 weist auf seiner Unterseite 35 eine Profilierung 36 auf. Die Profilierung 36 ist in der Form so gewählt, dass sie in die Aussparung 46 der Halterung 41 bzw. die Vertiefung 54 des Körperteils 53 des Entriegelungsknopfs 44 passt. Auf der Unterseite 35 des Verbindungsfortsatzes 34 sind Sägezähne 37 vorgesehen, welche mit der Rastnocke 56 des Entriegelungsknopfs 44 einrasten. Damit ist die Mischvorrichtung 30 nach dem Verbinden mit der ersten Spritzenanordnung 10 nur durch Drücken des Entriegelungsknopfs 44 lösbar.

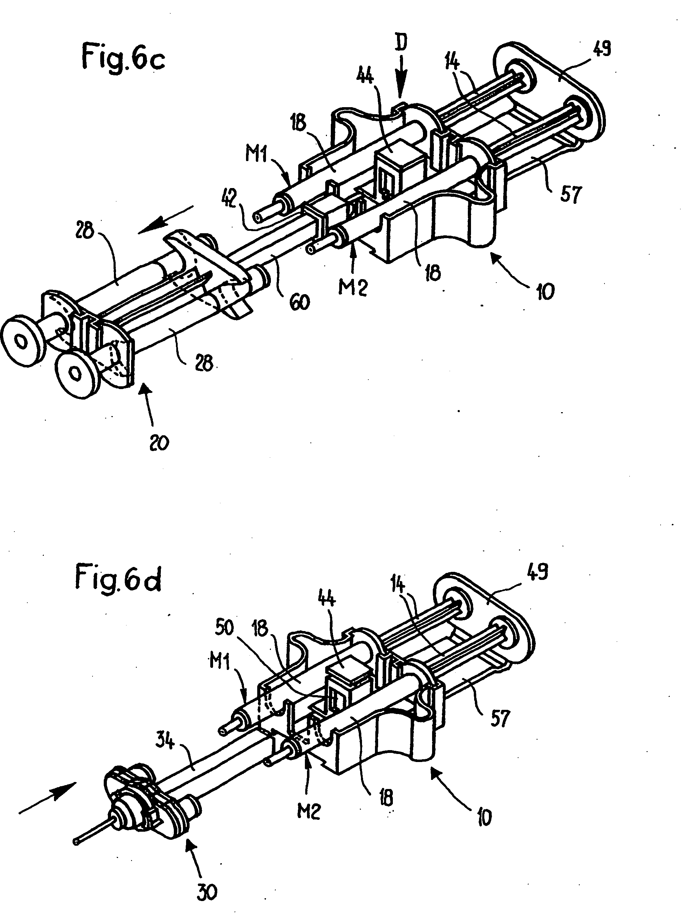

Der Mischvorgang ist nachfolgend in den Figuren 6a bis 6d gezeigt. Vor dem Mischen sind die zu vermischenden Komponenten in den Spritzen 28 der zweiten Spritzenanordnung 20 bzw. in den Spritzen 18 der ersten Spritzenanordnung 10 enthalten. Zum Mischen werden die beiden Spritzenanordnungen 10, 20 miteinander verbunden. Dazu wird der Verlängerungsfortsatz 60 in die Öffnung 45 der Codierhülse 42 der ersten Spritzenanordnung 10 eingefügt. Optional kann durch Wahl des Querschnittes des Verlängerungsfortsatzes 60 und der Öffnung 45 in der Codierhülse 42 sichergestellt werden, dass die beiden Spritzenanordnungen 10, 20 nur auf eine genau definierte Art und Weise zusammengesetzt werden können. In Figur 6b sind die zusammengesetzten Spritzenanordnungen 10, 20 gezeigt. Zum Vermischen der Komponenten wird die Verbindungsplatte 49 der ersten Spritzenanordnung 10 in Pfeilrichtung A gedrückt. Dadurch werden die in den Spritzen 18 enthaltenen Komponenten K1, K2 in die Spritzen 28 der zweiten Spritzenanordnung 20 transferiert. Damit werden die Kolben 24 der zweiten Spritzenanordnung 20 in die geöffnete Position gedrückt (siehe Figur 6b). In dieser Position lassen sich die Spritzenanordnungen 10, 20 nicht mehr voneinander trennen, da der Verlängerungsfortsatz 60 fest in der Codierhülse 42 eingerastet ist und sich der Entriegelungsknopf 44 zum Freigeben der Codierhülse 42 nicht nach unten bewegen lässt (siehe auch Figur 5a). In einem weiteren Schritt (zwischen Figur 6b und Figur 6c) werden die Kolben 24 der zweiten Spritzenanordnung betätigt. Dabei werden die in den Spritzen 28 der zweiten Spritzenanordnung 20 enthaltenen Gemische M1, M2 weiter gemischt und in die Spritzen 18 der ersten Spritzenanordnung 10 zurücktransferiert. Die Kolben 14 der ersten Spritzenanordnung 10 werden in die geöffnete Stellung gedrückt. Gleichzeitig wird die Verbindungsplatte 49 und dadurch auch die Verlängerung 57 verschoben. Auf diese Weise wird der Verriegelungsknopf 44 freigegeben (siehe Figur 5b), so dass er nun in Pfeilrichtung D nach unten gedrückt werden kann. Dadurch wird die Codierhülse 42 freigegeben und die zweite Spritzenanordnung 20 kann mit der auf dem Verbindungsfortsatz 60 verbleibenden Codierhülse 42 entfernt werden.

Zur Beendigung des Mischvorgangs wird die Mischvorrichtung 30 auf die erste Spritzenanordnung 10 aufgesetzt (siehe Figur 6d). Die Mischvorrichtung 30 weist ebenfalls einen Verbindungsfortsatz 34 auf (siehe auch Figur 5c). Der profilierte Verbindungsfortsatz 34 der Mischvorrichtung 30 ist in der Form so gewählt, dass er in die Öffnung 50 (mit der Aussparung 46) der Halterung 41 bzw. der Vertiefung 54 des Körperteils 53 des Entriegelungsknopfs 44 passt. Wie in Figur 5c dargestellt, sind auf der Unterseite 35 des Verbindungsfortsatzes 34 Sägezähne 37 vorgesehen, welche mit der Rastnocke 56 des Entriegelungsknopfs 44 einrasten. Damit ist die Mischvorrichtung 30 nach dem Verbinden mit der ersten Spritzenanordnung 10 nur durch Drücken des Entriegelungsknopfs 44 lösbar. Zur Mischung der Gemische M1, M2 in den Spritzen 18 der ersten Spritzenanordnung 10 wird wiederum die Verbindungsplatte 49 gedrückt. Dadurch werden die Kolben 14 betätigt und die Gemische M1, M2 durch die Mischvorrichtung 30 abgegeben. Nach der Abgabe bleibt die Mischvorrichtung 30 mit dem Verbindungsfortsatz 34 fest mit der ersten Spritzenanordnung 10 verbunden. In der Regel verbleibt die Mischvorrichtung 30 nach der Abgabe der Gemische an der ersten Spritzenanordnung 10. Allerdings ist die Mischvorrichtung 30 trotzdem im Falle von Störungen wie beispielsweise einer Verstopfung oder einer vorzeitigen Aushärtung des Gemisches aus M1 und M2 in der Mischvorrichtung 30 gegen einen Ersatz austauschbar.

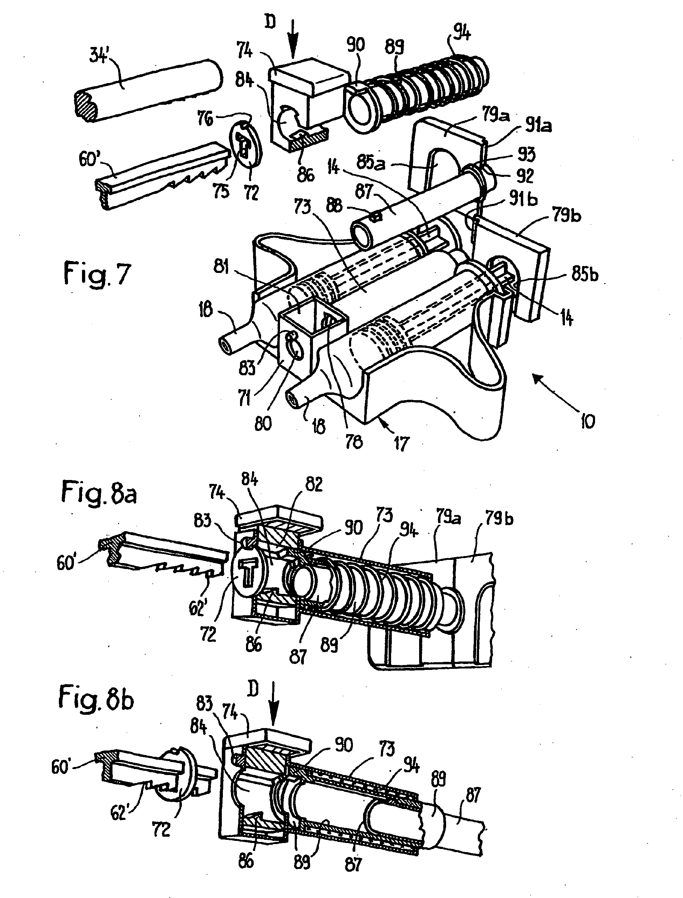

Figuren 7 bis 9 zeigen eine alternative Ausführungsform einer Verbindungsanordnung. Die Verbindungsanordnung gemäss Figuren 7 bis 9 hat eine ähnliche Funktion wie die Anordnung gemäss Figuren 4 bis 6 bei leicht unterschiedlicher Konstruktion.

Figur 7 zeigt die Explosionsdarstellung einer ersten Spritzenanordnung 10 gemäss dieser Ausführungsform. Ähnlich wie in vorhergehenden Ausführungsbeispielen besteht die erste Spritzenanordnung 10 aus einem Halter 17, in dem zwei Spritzen 18 angeordnet sind. Der Halter 17 ist mit einer Halterung 71 versehen. Die Halterung 71 dient zur Aufnahme einer Codierscheibe 72 und eines Entriegelungsknopfes 74. Dazu ist die Halterung 71 mit einer Längsöffnung 81 zur Aufnahme des Entriegelungsknopfes 74 versehen. Eine Öffnung 80 dient zur Aufnahme der Codierscheibe 72 sowie zur Aufnahme von Verbindungsfortsätzen 60' bzw. 34' einer zweiten spritzenanordnung 20 bzw. einer Mischvorrichtung 30. An der Halterung 71 ist ein Zapfen 83 vorgesehen, der in eine Nut 76 der Codierscheibe 72 passt. Dadurch ist die Lage der Codierscheibe 72 genau definiert. Die Codierscheibe 72 weist eine Öffnung 75 mit T-förmigem Querschnitt auf. Der Verbindungsfortsatz 60' weist ebenfalls einen T-förmigen Querschnitt auf, so dass die mit einem T-förmigen Verbindungsfortsatz 60' verbundene zweite Spritzenanordnung 20 nur in einer genau definierten Lage mit der ersten Spritzenanordnung 10 verbunden werden kann. Anschliessend an die Halterung 71 ist eine zylindrische Führung 73 in dem Halter 17 angeordnet. Die zylindrische Führung 73 dient zur Aufnahme eines Verriegelungsrohrs 89. Das Verriegelungsrohr 89 weist einen Vorsprung 90 auf. Der Entriegelungsknopf 74 ist in Pfeilrichtung D (siehe Figur 8b) verschiebbar in der Längsöffnung 81 gehalten. Der Entriegelungsknopf 74 weist selbst eine Öffnung 84 zur Aufnahme des Verbindungsfortsatzes 60' auf. Eine Rastnocke 86 in der Öffnung 84 des Entriegelungsknopfes 74 dient zum rastenden Verbinden mit Sägezähnen 62' des Verbindungsfortsatzes 60'. Wenn sich das Verriegelungsrohr 89 in der Figur 8a gezeigten Stellung befindet, untergreift der Vorsprung 90 die obere Begrenzungsfläche 82 der Öffnung 84 des Entriegelungsknopfes 74. Der Entriegelungsknopf 74 kann daher nicht in Pfeilrichtung D (siehe Figur 8b) gedrückt werden. Daher kann der in die Öffnung 84 eingesetzte Verbindungsfortsatz 60' in der in Figur 8a gezeigten Stellung nicht entfernt werden. Die Öffnung 84 ist weiterhin so ausgebildet, dass eine formschlüssige Aufnahme des Verbindungsfortsatzes 34' möglich ist. Der Verbindungsfortsatz 34' weist ebenfalls Sägezähne auf, die mit der Rastnocke 86 in Eingriff gelangen.

Innerhalb des Verriegelungsrohrs 89 ist ein Betätigungsstück 87 angeordnet (siehe auch Figur 8b). Das Betätigungsstück 87 ist mit Clips 79a, 79b verbindbar. Die Clips 79a, 79b sind senkrecht zur Verschieberichtung der Kolben 14 verschiebbar, so dass sie in Art einer Feder und Nut miteinander verbindbar sind. Die Clips 79a, 79b sind dabei mit einer Längsaussparung 85a, 85b auf die Enden der Kolben 14 aufschiebbar. Auf den einander zugewandten Seiten 91a, 91b sind die Clips 79a, 79b mit einer Öffnung 92 versehen. Die Öffnung 92 dient zur rastenden Aufnahme eines Rastvorsprungs 93 an dem Betätigungsstück 87.

In zusammengesetztem Zustand ist das Betätigungsstück 87 im Verriegelungsrohr 89 angeordnet. Das Verriegelungsrohr 89 seinerseits ist in der zylindrischen Führung 73 in Achsrichtung verschiebbar gehalten. Das Verriegelungsrohr 89 wird über eine Feder 94 gespannt. Am Betätigungsstück 87 ist ein Nocken 88 vorgesehen, der in einer Nut (nicht dargestellt) des Verriegelungsrohrs 89 geführt wird. Dadurch wird zum einen sicherstellt, dass zur Entfernung des Verbindungsfortsatzes 60' der Vorsprung 90 gegen die Federkraft der Feder 94 aus einer in der Halterung 71 vorhandenen Zentrieröffnung 78 entfernt werden kann und so die obere Begrenzungsfläche 82 der Öffnung 84 des Entriegelungsknopfes 74 freigegeben wird. Zum anderen lässt sich das Verriegelungsrohr 89 und damit der Vorsprung 90 aufgrund der Nut-Nocken-Kombination mit dem Betätigungsstück 87 gegenüber der Zentrieröffnung 78 ausrichten. Eine Blockade des Entriegelungsknopfes 74 durch den Vorsprung 90 ist nur möglich, wenn der Vorsprung 90 durch die Zentrieröffnung 78 führbar ist.

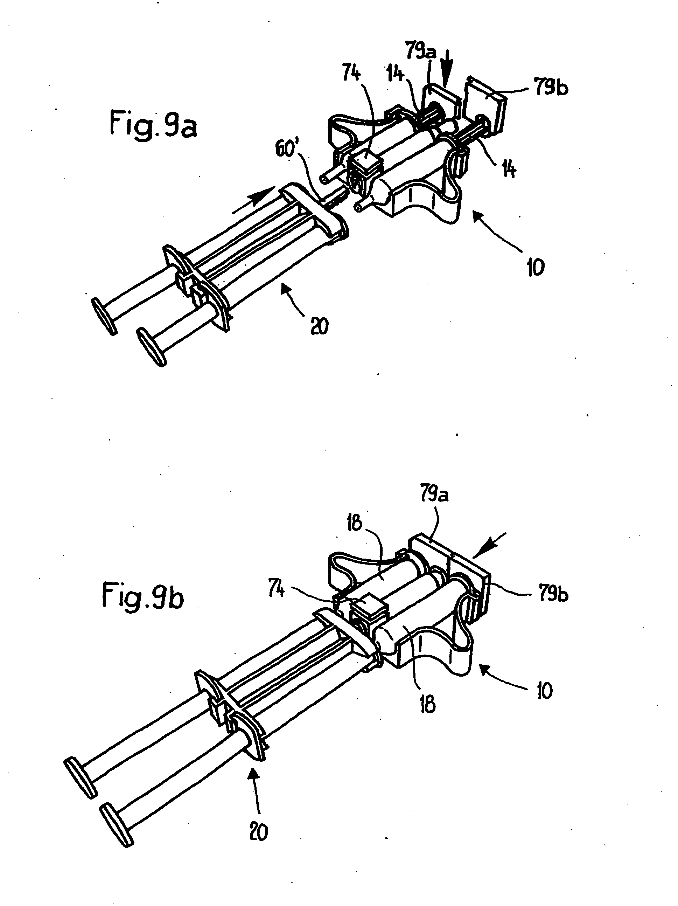

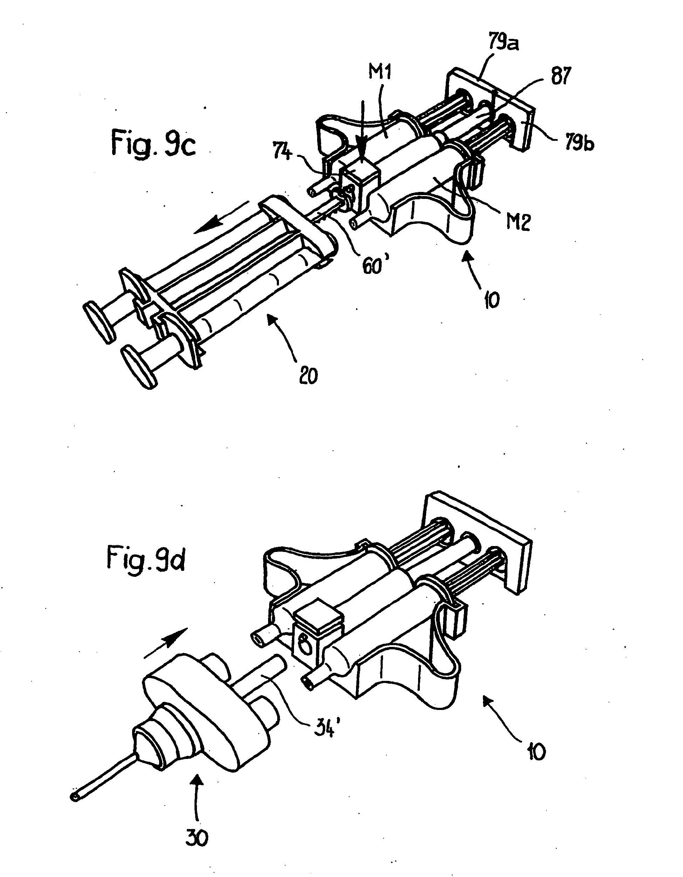

Die erste und die zweite Spritzenanordnung 10, 20 werden danach so miteinander verbunden, dass der Verbindungsfortsatz 60' durch die Codierscheibe 72 in die Öffnung 84 des Entriegelungsknopfes 74 gelangt, und dass die Sägezähne 62' mit dem Rastnocken 86 verrasten (siehe Figur 9a). Danach erfolgt die Vermischung der Komponenten durch Betätigen der Kolben der Spritzen 18 der ersten Spritzenanordnung 10 (siehe Figur 9b). Sobald die Enden der Kolben sich auf gleicher Höhe befinden, können die beiden Clips 79a, 79b auf die Kolben 14 aufgesetzt und miteinander verbunden werden (siehe Figur 9a). In der in Figur 9b gezeigten Situation (siehe auch Figur 8a) liegt der Vorsprung 90 des Verriegelungsrohrs 89 unter der oberen Begrenzungsfläche 82 der Öffnung 84 des Entriegelungsknopfes 74. Dadurch kann die Verbindung nicht entriegelt werden und die Spritzenanordnungen 10, 20 können nicht voneinander getrennt werden. Beim Betätigen der Kolben 14 der Spritzen der ersten Spritzenanordnung 10 wird das Betätigungsstück 87 mit den Clips 79a, 79b verbunden. Durch Zurückziehen der Kolben 14 (siehe Figur 9c) wird auch das Betätigungsstück 87 wieder zurückgezogen. Dadurch zieht sich auch das Verriegelungsrohr 89 zurück und gibt die Begrenzungsfläche 82 des Entriegelungsknopfs 74 frei (siehe Figur 8a und 9c). Durch Drücken des Verriegelungsknopfs 74 lässt sich wie in Figur 9c gezeigt, die zweite Spritzenanordnung 20 von der ersten Spritzenanordnung 10 entfernen. Die Codierscheibe 72 bleibt dabei auf dem Verbindungsfortsatz 60'.

Auf diese Weise ist sichergestellt, dass die Spritzenanordnungen 10, 20 erst dann voneinander getrennt werden können, wenn durch Hin- und Hertransfer die Gemische M1, M2 sich in den Spritzen 18 der ersten Spritzenanordnung 10 befinden. Wenn der Transfer nur von der zweiten Spritzenanordnung 20 in die erste Spritzenanordnung 10 geschehen würde, würde das Verriegelungsrohr 89 nicht zurückgezogen, weil ursprünglich keine Verbindung zwischen den Kolben 14 und dem Verriegelungsrohr bzw. dem Betätigungsstück 87 besteht. Wenn der Transfer nur von der ersten Spritzenanordnung 10 in die zweite Spritzenanordnung 20 und nicht zurück erfolgt, kann der Entriegelungsknopf 74 ebenfalls nicht betätigt werden.

Im letzten in Figur 9d gezeigten Schritt (siehe auch Figur 8b) wird die Mischvorrichtung 30 ähnlich wie in Figur 6d gezeigt mit der ersten Spritzenanordnung 10 verbunden. Der Verbindungsfortsatz 34' greift in die Öffnung 84 des Betätigungsknopfes 74 ein. Die Sägezähne 37' verrasten dabei mit dem Rastnocken 86.

## Patentansprüche

1. Vorrichtung zum Mischen von wenigstens vier Komponenten (K1, K2, K3, K4) mit einer ersten Spritzenanordnung (10) und mit einer zweiten Spritzenanordnung (20), wobei in der ersten und in der zweiten Spritzenanordnung (10, 20) je wenigstens zwei Kammern (11, 12; 21, 22) zur Aufnahme je einer der Komponenten (K1, K2, K3, K4) enthalten sind, wobei jede Kammer (11, 12) der einen Spritzenanordnung (10) lösbar mit einer Kammer (21, 22) der anderen Spritzenanordnung (20) verbindbar ist, so dass jede in einer Kammer (11, 12) der einen Spritzenanordnung (10) enthaltene Komponente (K1, K2) durch Transfer in eine Kammer (21, 22) der anderen Spritzenanordnung (20) mit der in dieser Kammer (21 22) enthaltenen Komponente (K3, K4) mischbar ist, **dadurch gekennzeichnet, dass** die beiden Spritzenanordnungen (10, 20) mit Codiermitteln (42, 60, 72, 60') in Form einer Codierhülse (42) oder einer Codierscheibe (72) sowie einem zugeordneten Verbindungsfortsatz (60, 60') ausgestattet sind, um sicherzustellen, dass die beiden Spritzenanordnungen (10, 20) nur auf eine genau definierte Art und Weise zusammenfügbar sind.

2. Vorrichtung nach Anspruch 1, **dadurch gekennzeichnet, dass** die Codierhülse (42) bzw. die Codierscheibe (72) eine Öffnung (45, 75) mit einem Querschnitt aufweist, in welche der Verbindungsfortsatz (60, 60') mit einem dazu gleichartigen Querschnitt nur in einer genau definierten Lage der Spritzenanordnungen (10, 20) in Eingriff gelangt.

3. Vorrichtung nach Anspruch 1 oder 2, **dadurch gekennzeichnet, dass** die Spritzenanordnung (10, 20) mit einer Einrastanordnung ausgestattet ist, die einen Entriegelungsknopf (44, 74) und eine damit derart zusammenwirkbare Verlängerung (57) bzw. ein derart damit zusammenwirkbares Verriegelungsrohr (89) aufweist, dass sichergestellt ist, dass die Spritzenanordnungen (10, 20) erst dann voneinander getrennt werden können, wenn sich die vermischten Komponenten (K1, K2, K3, K4) in der ersten Spritzenanordnung (10) befinden.

4. Vorrichtung nach einem der Ansprüche 1 bis 3, **dadurch gekennzeichnet, dass** die Verbindung zwischen der ersten und der zweiten Spritzenanordnung (10, 20) nur unter Entfernung der auf dem Verbindungsfortsatz (60, 60') verbleibenden Codierhülse (42) bzw. Codierscheibe (72) lösbar ist.

5. Vorrichtung nach einem der Ansprüche 1 bis 4, **dadurch gekennzeichnet, dass** die Vorrichtung eine Mischanordnung (30), vorzugsweise einen statischen Mischer aufweist, die mit den Kammern (11, 12) der ersten Spritzenanordnung (10) verbindbar ist, so dass in den Kammern (11, 12) enthaltenen erste Gemische (M1, M2) aus den Komponenten (K1, K2 bzw. K3, K4) durch Abgabe aus den Kammern (11, 12) durch die Mischanordnung (30) weiter miteinander zu einem zweiten Gemisch (M3) mischbar sind.

6. Vorrichtung nach Anspruch 4 und 5, **dadurch gekennzeichnet, dass** die Mischanordnung (30) erst dann mit den Kammern (11, 12) der ersten Spritzenanordnung (10) verbindbar ist, wenn die Codierhülse (42) bzw. Codierscheibe (72) gelöst worden ist.

7. Vorrichtung nach einem der Ansprüche 1 bis 6, **dadurch gekennzeichnet, dass** wenigstens eine Spritzanordnung (10, 20) betreffende Kammern (11, 12; 21, 22) bildende Spritzen (18, 28) mit Spritzenkolben (14, 24) aufweist, welche miteinander, vorzugsweise mittels eines Clips verbindbar sind.

8. Vorrichtung nach einem der Ansprüche 1 bis 6, **dadurch gekennzeichnet, dass** die eine Spritzenanordnung (10) Spritzen (18) aus Glas und die andere Spritzenanordnung (20) Spritzen (28) aus Kunststoff aufweist, wobei die Spritzen (18, 28) die betreffenden Kammern (11, 12; 21, 22) bilden.

9. Vorrichtung nach Anspruch 8, **dadurch gekennzeichnet, dass** in den Kammern (11, 12) der aus Glas gebildeten Spritzenanordnung (10) PEG-Acrylate oder PEG-Thiol und in den Kammern (21, 22) der aus Kunststoff gebildeten Spritzenanordnung (20) Puffer enthalten sind.

10. Vorrichtung nach Anspruch 8 oder 9, **dadurch gekennzeichnet, dass** wenigstens die Spritzen (18, 28) der einen Spritzanordnung (10, 20) miteinander, vorzugsweise mittels eines Clips verbindbar sind.

11. Vorrichtung zum Aufbringen einer Mischung aus zwei Komponenten, umfassend eine erste Spritzenanordnung (10) und eine Mischanordnung (30), **dadurch gekennzeichnet, dass** die erste Spritzenanordnung (10) ein Codiermittel in Form einer Codierhülse (42) oder einer Codierscheibe (72) aufweist und mit einer Öffnung (50, 84) ausgestattet ist, in welche ein Verbindungsfortsatz (34, 34') der Mischvorrichtung (30) passt.

12. Kit umfassend eine Vorrichtung nach einem der Ansprüche 1 bis 10 zum Mischen von wenigstens vier Komponenten (K1, K2, K3, K4) mit einer ersten Spritzenanordnung (10) und mit einer zweiten Spritzenanordnung (20) und eine Mischanordnung (30), **dadurch gekennzeichnet, dass** die erste Spritzenanordnung (10) ein Codiermittel in Form einer Codierhülse (42) oder einer Codierscheibe (72) aufweist und dass die erste Spritzenanordnung (10) entweder eindeutig mit der zweiten Spritzenanordnung (20) oder nach Entfernung der Codierhülse (42) oder der Codierscheibe (72) mit der Mischanordnung (30) über deren Verbindungsfortsatz (34, 34') verbindbar ist.

13. Halter, wobei der Halter (17) zum Bilden einer ersten Spritzenanordnung (10) zur Aufnahme von wenigsten zwei Spritzen (18, 28) ausgebildet ist, **dadurch gekennzeichnet, dass** der Halter (17) mit Codiermitteln (42, 60, 72, 60') in Form einer Codierhülse (42) oder einer Codierscheibe (72) ausgestattet ist, um sicherzustellen, dass die erste Spritzenanordnung (10) und eine zweite Spritzenanordnung (20) nur auf eine genau definierte Art und Weise derart lösbar zusammensetzbar sind, dass Ausgabeöffnungen (16a, 16b) der Spritzen (18) der ersten Spritzenanordnung (10) mit Öffnungen (26a, 26b; 16a, 16b) von Spritzen (18, 28) der zweiten Spritzenanordnung (20) auf eine genau definierte Art dichtend verbindbar sind oder dass Ausgabeöffnungen (16a, 16b) der Spritzen (18) der ersten Spritzenanordnung (10) mit Öffnungen (32) einer Mischanordnung (30) nach Entfernung der Codierhülse (42) oder der Codierscheibe (72) dichtend verbindbar sind.

14. Verfahren zum Mischen von wenigstens vier Komponenten (K1, K2, K3, K4) aufweisend die Schritte:
- Bereitstellen von Komponenten (K1, K2, K3, K4) in je wenigstens zwei Kammern (11, 12; 21, 22) von einer ersten Spritzenanordnung (10) und einer zweiten Spritzenanordnung (20), welche Kammern (11, 12; 21, 22) je mit Ausgabeöffnungen (16a, 16b, 26a, 26b) versehen sind, **gekennzeichnet durch** die weiteren Schritte
- eindeutiges Verbinden der Ausgabeöffnungen (16a, 16b) je einer Kammer (11, 12) der ersten Spritzenanordnung (10) mit je einer Ausgabeöffnung (26a, 26b) einer Kammer (21, 22) der zweiten Spritzenanordnung (20) auf eine genau definierte Art, wobei die Spritzenanordnungen (10, 20) mit Codiermitteln (42, 60, 72, 60') in Form einer Codierhülse (42) oder einer Codierscheibe (72) sowie einem zugeordneten Verbindungsfortsatz (60, 60') ausgestattet sind;
- Transferieren der Komponenten (K1, K2) aus den Kammern (11, 12) der ersten Spritzenanordnung (10) in je eine vorbestimmte Kammer (21, 22) der zweiten Spritzenanordnung (20), wobei die transferierten Komponenten (K1, K2) mit den in diesen Kammern (21, 22) enthaltenen Komponenten (K3, K4) zu ersten Gemischen (M1, M2) vermischt werden;
- Rücktransfer der Gemische (M1, M2) zwecks weiterer Vermischung aus den Kammern (21, 22) der zweiten Spritzenanordnung (20) in die Kammern (11, 12) der ersten Spritzenanordnung (10);
- Trennung der ersten und zweiten Spritzenanordnung (10,20) unter Entfernung der Codierhülse (42) oder der Codierscheibe (72);
- Aufsetzen einer Mischanordnung (30) auf die erste Spritzenanordnung (10);
- Abgabe der in den Kammern (11, 12; 21, 22) enthaltenen Gemische (M1, M2) unter weiterer Mischung zur Bildung eines zweiten Gemisches (M3) aus allen Komponenten (K1, K2, K3, K4).

## Claims

1. Device for mixing at least four components (K1, K2, K3, K4) having a first syringe arrangement (10) and having a second syringe arrangement (20), the first and the second syringe arrangements (10, 20) each containing at least two chambers (11, 12; 21, 22) for holding one each of the components (K1, K2, K3, K4), it being possible to connect each chamber (11, 12) of one of the syringe arrangements (10) in a detachable fashion to a chamber (21, 22) of the other syringe arrangement (20) so that each component (K1, K2) which is contained in a chamber (11, 12) of one of the syringe arrangements (10) can be mixed, by transferring into a chamber (21, 22) of the other syringe arrangement (20), with the component (K3, K4) which is contained in this chamber (21, 22), **characterized in that** the two syringe arrangements (10, 20) are equipped with coding means (42, 60, 72, 60') in the form of a coding sleeve (42) or a coding disc (72) as well as an assigned connecting projection (60, 60'), in order to ensure that the two syringe arrangements (10, 20) can only be joined in a precisely defined way.

2. Device according to Claim 1, **characterized in that** the coding sleeve (42) or the coding disc (72) has an opening (45, 75) with a cross section, into which opening (45, 75) the connecting projection (60, 60') engages with an identical cross section only in a precisely defined position of the syringe arrangements (10, 20).

3. Device according to Claim 1 or 2, **characterized in that** the syringe arrangement (10, 20) is equipped with a latching arrangement which has an unlocking knob (44, 74) and an extension (57) which can interact therewith or has a locking pipe (69) which can interact therewith in such a way that it is ensured that the syringe arrangements (10, 20) cannot be separated from one another until the mixed components (K1, K2, K3, K4) are located in the first syringe arrangement (10).

4. Device according to one of Claims 1 to 3, **characterized in that** the connection between the first and second syringe arrangements (10, 20) can be released only by removing the coding sleeve (42) or coding disc (72) which remains on the connecting projection (60, 60').

5. Device according to one of Claims 1 to 4, **characterized in that** the device has a mixing arrangement (30), preferably a static mixer, which can be connected to the chambers (11, 12) of the first syringe arrangement (10) so that first mixtures (M1, M2) of the components (K1, K2 or K3, K4) which are contained in the chambers (11, 12) can be mixed further with one another to form a second mixture (M3) by being discharged from the chambers (11, 12) through the mixing arrangement (30).

6. Device according to Claims 4 and 5, **characterized in that** the mixing arrangement (30) cannot be connected to the chambers (11, 12) of the first syringe arrangement (10) until the coding sleeve (42) or coding disc (72) has been released.

7. Device according to one of Claims 1 to 6, **characterized in that** at least one syringe arrangement (10, 20) has syringes (18, 28), which form chambers (11, 12; 21, 22) in question and have syringe plungers (14, 24) which can be connected to one another, preferably by means of a clip.

8. Device according to one of Claims 1 to 6, **characterized in that** one of the syringe arrangements (10) has syringes (18) made of glass, and/or the other syringe arrangement (20) has syringes (28) made of plastic, the syringes (18, 28) forming the chambers (11, 12; 21, 22) in question.

9. Device according to Claim 8, **characterized in that** PEG acrylates or PEG thiol are contained in the chambers (11, 12) of the syringe arrangement (10) which is formed from glass, and buffers are contained in the chambers (21, 22) of the syringe arrangement (20) which is formed from plastic.

10. Device according to Claim 8 or 9, **characterized in that** at least the syringes (18, 28) of one of the syringe arrangements (10, 20) can be connected to one another, preferably by means of a clip.

11. Device for forming a mixture of two components, comprising a first syringe arrangement (10) and a mixing arrangement (30), **characterized in that** the first syringe arrangement (10) has a coding means in the form of a coding sleeve (42) or a coding disc (72), and is equipped with an opening (50, 84) into which a connecting projection (34, 34') of the mixing device (30) fits.

12. Kit comprising a device according to one of Claims 1 to 10 for mixing at least four components (K1, K2, K3, K4) with a first syringe arrangement (10) and with a second syringe arrangement (20) and a mixing arrangement (30), **characterized in that** the first syringe arrangement (10) has a coding means in the form of a coding sleeve (42) or a coding disc (72), and **in that** the first syringe arrangement (10) can either be connected uniquely to the second syringe arrangement (20), or can be connected to the mixing arrangement (30) via its connecting projection (34, 34') after removal of the coding sleeve (42) or the coding disc (72).

13. Holder, the holder (17) being designed to form a first syringe arrangement (10) for holding at least two syringes (18, 28), **characterized in that** the holder (17) is equipped with coding means (42, 60, 72, 60') in the form of a coding sleeve (42) or a coding disc (72) in order to ensure that the first syringe arrangement (10) and a second syringe arrangement (20) can be releaseably joined only in a precisely defined way such that discharge openings (16a, 16b) of the syringes (18) of the first syringe arrangement (10) can be connected in a seal-forming way to openings (26a, 26b; 16a, 16b) of syringes (18, 28) of the second syringe arrangement (20) in a precisely defined way, or **in that** discharge openings (16a, 16b) of the syringes (18) of the first syringe arrangement (10) can be connected in a seal-forming way to openings (32) of a mixing arrangement (30) after removal of the coding sleeve (42) or the coding disc (72).

14. Method for mixing at least four components (K1, K2, K3, K4) having the following steps:
- components (K1, K2, K3, K4) are provided in each case in at least two chambers (11, 12; 21, 22) of a first syringe arrangement (10) and of a second syringe arrangement (20), which chambers (11, 12; 21, 22) are each provided with discharge openings (16a, 16b, 26a, 26b), **characterized by** the further steps
- the discharge openings (16a, 16b) of in each case one chamber (11, 12) of the first syringe arrangement (10) are uniquely connected in each case to one discharge opening (26a, 26b) of a chamber (21, 22) of the second syringe arrangement (20) in a precisely defined way, wherein the syringe arrangements (10, 20) are equipped with coding means (42, 60, 72, 60') in the form of a coding sleeve (42) or a coding disc (72) and an assigned connecting projection (60, 60');
- the components (K1, K2) are transferred in each case from the chambers (11, 12) of the first syringe arrangement (10) into a predefined chamber (21, 22) of the second syringe arrangement (20), the transferred components (K1, K2) being mixed with the components (K3, K4) contained in these chambers (21, 22), to form first mixtures (M1, M2),
- the mixtures (M1, M2) are transferred back from the chambers (21, 22) of the second syringe arrangement (20) into the chambers (11, 12) of the first syringe arrangement (10) for the purpose of further mixing,
- the first and second syringe arrangements (10, 20) one separated by removal of the coding sleeve (42) or the coding disc (72);
- a mixing arrangement (30) is fitted onto the first syringe arrangement (10);
- the mixtures (M1, M2) contained in the chambers (11, 12; 21, 22) are discharged, accompanied by further mixing, in order to form a second mixture (M3) composed of all the components (K1, K2, K3, K4).

## Revendications

1. Dispositif pour mélanger au moins quatre composants (K1, K2, K3, K4) avec un premier arrangement de seringues (10) et avec un second arrangement de seringues (20), le premier et le second arrangement de seringues (10, 20) comportant chacun au moins deux chambres (11, 12; 21, 22), chaque chambre recevant l'un des composants (K1, K2, K3, K4), chaque chambre (11, 12) de l'un des arrangements de seringues (10) pouvant être reliée de manière détachable avec une chambre (21, 22) de l'autre arrangement de seringues (20), de sorte que chaque composant (K1, K2) contenu dans une des chambres (11, 12) de l'un des arrangements de seringues (10) peut être mélangé, par transfert dans une chambre (21, 22) de l'autre arrangement de seringues (20), avec le composant (K3, K4) contenu dans cette chambre (21, 22), **caractérisé en ce que** les deux arrangements de seringues (10, 20) sont pourvus de moyens de codage (42, 60, 72, 60') sous la forme d'une douille de codage (42) ou d'un disque de codage (72), ainsi que d'une prolongation de liaison associée (60, 60') afin de garantir que les deux arrangements de seringues (10, 20) ne puissent être assemblés que d'une manière exactement définie.

2. Dispositif selon la revendication 1, **caractérisé en ce que** la douille de codage (42) ou le disque de codage (72) présente une ouverture (45, 75) avec une section transversale dans laquelle la prolongation de liaison (60, 60'), qui a une section transversale correspondant à celle-ci, ne peut être mise en prise que dans une position des arrangements de seringues (10, 20) exactement définie.

3. Dispositif selon la revendication 1 ou 2, **caractérisé en ce que** l'arrangement de seringues (10, 20) est pourvu d'un dispositif d'enclenchement muni d'un bouton de déverrouillage (44, 74) et d'un prolongement (57) coopérant avec celui-ci ou d'un tube de verrouillage (89) coopérant avec celui-ci de manière à garantir que les arrangements de seringues (10, 20) ne puissent être séparés l'un de l'autre que lorsque les composants (K1, K2, K3, K4) mélangés se trouvent dans le premier arrangement de seringues (10).

4. Dispositif selon l'une des revendications 1 à 3, **caractérisé en ce que** la liaison entre le premier et le second arrangement de seringues (10, 20) ne peut être défaite qu'en retirant la douille de codage (42) ou le disque de codage (72) qui reste sur la prolongation de liaison (60, 60').

5. Dispositif selon l'une des revendications 1 à 4, **caractérisé en ce que** le dispositif est pourvu d'un dispositif de mélange (30), de préférence un mélangeur statique, qui peut être relié aux chambres (11, 12) du premier arrangement de seringues (10), de sorte que les premiers mélanges (M1, M2) des composants (K1, K2 respectivement K3, K4) contenus dans les chambres (11, 12) peuvent être à leur tour mélangés entre eux par leur expulsion des chambres (11, 12) à travers le dispositif de mélange (30), pour former un second mélange (M3).

6. Dispositif selon les revendications 4 et 5, **caractérisé en ce que** le dispositif de mélange (30) ne peut être relié avec les chambres (11, 12) du premier arrangement de seringues (10) que lorsque la douille de codage (42) ou le disque de codage (72) a été enlevé.

7. Dispositif selon l'une des revendications 1 à 6, **caractérisé en ce qu'**au moins un arrangement de seringues (10, 20) comporte des seringues (18, 28), avec des pistons de seringue (14, 24), formant les chambres (11, 12; 21, 22) en question et qui peuvent être reliées l'une avec l'autre, de préférence au moyen d'un clip.

8. Dispositif selon l'une des revendications 1 à 6, **caractérisé en ce que** l'un des arrangements de seringues (10) est pourvu de seringues (18) en verre et que l'autre arrangement de seringues (20) est pourvu de seringues (28) en matière plastique, les seringues (18, 28) formant les chambres (11, 12; 21, 22) en question.

9. Dispositif selon la revendication 8, **caractérisé en ce que** les chambres (11, 12) de l'arrangement de seringues (10) réalisé en verre contiennent des acrylates de PEG ou du thiol de PEG, et **en ce que** les chambres (21, 22) de l'arrangement de seringues (20) réalisé en matière plastique contiennent des tampons.

10. Dispositif selon la revendication 8 ou 9, **caractérisé en ce qu'**au moins les seringues (18, 28) de l'un des arrangements de seringues (10, 20) peuvent être reliées l'une avec l'autre, de préférence au moyen d'un clip.

11. Dispositif pour l'application d'un mélange de deux composants comprenant un premier arrangement de seringues (10) et un dispositif de mélange (30), **caractérisé en ce que** le premier arrangement de seringues (10) est pourvu d'un moyen de codage sous la forme d'une douille de codage (42) ou d'un disque de codage (72) et est muni d'une ouverture (50, 84) dans laquelle s'adapte une prolongation de liaison (34, 34') du dispositif de mélange (30).

12. Kit comportant un dispositif selon l'une des revendications 1 à 10 pour mélanger au moins quatre composants (K1, K2, K3, K4) avec un premier arrangement de seringues (10) et avec un second arrangement de seringues (20) et un dispositif de mélange (30), **caractérisé en ce que** le premier arrangement de seringues (10) est pourvu d'un moyen de codage sous la forme d'une douille de codage (42) ou d'un disque de codage (72) et **en ce que** le premier arrangement de seringues (10) peut être relié de manière à exclure toute erreur soit avec le second arrangement de seringues (20) soit, après enlèvement de la douille de codage (42) ou du disque de codage (72), avec le dispositif de mélange (30), par l'intermédiaire de la prolongation de liaison (34, 34') de celui-ci.

13. Support, le support (17) étant configuré, pour former un premier arrangement de seringues (10), de sorte à recevoir au moins deux seringues (18, 28), **caractérisé en ce que** le support (17) est équipé de moyens de codage (42, 60, 72, 60') sous la forme d'une douille de codage (42) ou d'un disque de codage (72), de manière à garantir que le premier arrangement de seringues (10) et un second arrangement de seringues (20) puissent être assemblés de manière exactement définie et détachable, afin de permettre une liaison étanche, de manière exactement définie, entre les ouvertures de sortie (16a, 16b) des seringues (18) du premier arrangement de seringues (10) et les ouvertures (26a, 26b; 16a, 16b) des seringues (18, 28) du second arrangement de seringues (20), ou afin de permettre une liaison étanche entre les ouvertures de sortie (16a, 16b) des seringues (18) du premier arrangement de seringues (10) et les ouvertures (32) d'un dispositif de mélange (30) après enlèvement de la douille de codage (42) ou du disque de codage (72).

14. Procédé pour mélanger au moins quatre composants (K1, K2, K3, K4), présentant les étapes suivantes:
- fourniture de composants (K1, K2, K3, K4) logés respectivement dans au moins deux chambres (11, 12; 21, 22) d'un premier arrangement de seringues (10) et d'un second arrangement de seringues (20), lesquelles chambres (11, 12; 21, 22) sont pourvues chacune d'une ouverture de sortie (16a, 16b; 26a, 26b), **caractérisée par** les étapes suivantes
- liaison excluant toute erreur, de manière exactement définie, des ouvertures de sortie (16a, 16b) respectivement d'une chambre (11, 12) du premier arrangement de seringues (10) avec respectivement une ouverture de sortie (26a, 26b) d'une chambre (21, 22) du second arrangement de seringues (20), les arrangements de seringues (10, 20) étant pourvus de moyens de codage (42, 60, 72, 60') sous la forme d'une douille de codage (42) ou d'un disque de codage (72), ainsi que d'une prolongation de liaison associée (60, 60') ;
- transfert des composants (K1, K2) des chambres (11, 12) du premier arrangement de seringues (10) respectivement dans une chambre (21, 22) prédéterminée du second arrangement de seringues (20), les composants (K1, K2) transférés étant mélangés avec les composants (K3, K4) contenus dans ces chambres (21, 22) pour former des premiers mélanges (M1, M2);
- transfert inverse des mélanges (M1, M2), dans le but d'un mélange supplémentaire, des chambres (21, 22) du second arrangement de seringues (20) dans les chambres (11, 12) du premier arrangement de seringues (10) ;
- séparation du premier et du second arrangement de seringues (10, 20), avec enlèvement de la douille de codage (42) ou du disque de codage (72) ;
- mise en place d'un dispositif de mélange (30) sur le premier arrangement de seringues (10) ;
- expulsion des mélanges (M1, M2) contenus dans les chambres (11, 12; 21, 22) avec réalisation d'un mélange supplémentaire pour former un second mélange (M3) de tous les composants (K1, K2, K3, K4).
